# EUROPEAN PATENT SPECIFICATION

(11) **EP 0 769 018 B1**
(45) Date of publication and mention of the grant of the patent: **18.12.2002**
(21) Application number: 95922708.3
(22) Date of filing: 03.07.1995
(51) Int. Cl.: C07K 14/205, C07K 16/12, A61K 39/106, A61K 39/40

(54) **HELICOBACTER PROTEINS AND VACCINES**
HELICOBACTER PROTEINE UND IMPSTOFFE
PROTEINES D'HELICOBACTER ET VACCINS

(30) Priority: 01.07.1994 IE 940538; 06.04.1995 IE 950249
(43) Date of publication of application: 23.04.1997
(73) Proprietor: CHIRON CORPORATION, Emeryville, California 94608 (US)
(72) Inventor: KELLEHER, Dermot, Dun Laoghaire County Dublin (IE); WINDLE, Henry, Dublin 4 (IE); BYRNE, William, Mount Merrion County Dublin (IE); McMANUS, Ross, Dublin 2 (IE)
(74) Representative: Carpmaels & Ransford
(86) International application number: IE9500036
(87) International publication number: WO96001272

(56) References cited:
- WO-A-93/18150
- JOURNAL OF BACTERIOLOGY, vol. 173, no. 2, January 1991 pages 505-513, O'TOOLE P.W. ET AL. 'Isolation and Biochemical and Molecular Analyses of a Species-Specific Protein Antigen from the Gastric Pathogen Helicobacter pylori'
- GUT, vol. 35, 1994 pages 1379-1384, FAN X.J. ET AL. 'Gastric T lymphocytes responses to Helicobacter pylori patients with H pylori colonisation' cited in the application

## Description

### Field of the Invention

The invention relates to a vaccine or therapeutic composition for the treatment or prophylaxis of *Helicobacter pylori* associated disease and protein used in the vaccine.

### Background

*Helicobacter pylori* is a widely prevalent organism found on gastric biopsy in approximately 30% of the population less than 40 years old with increasing incidence thereafter. The organism is a causative agent of chronic gastritis in humans (e.g. Marshall & Warren 1984¹; Blaser, 1990²). Epidemiological studies have shown that *H*. *pylori* is most commonly found in association with gastritis. Serological investigations have demonstrated that evidence of a current or prior infection can be found in 30 - 50% of a randomly chosen population of blood donors. No direct causal relationship has been conclusively proven for duodenal ulcer disease. However, the organism is found in 95% of patients with duodenal ulcer. Furthermore, eradication of the organism results in rapid ulcer healing (e.g. Rauws & Tytgat, 1990³). These data provide strong evidence that *H*. *pylori* is a dominant factor in the development of duodenal ulcer. Additional evidence for the pathogenic involvement of *H. pylori* in these conditions has been provided by studies with gnotobiotic piglets (Lambert et al., 1987⁴) and the fulfilment of Koch's postulates with human volunteers (Marshall et al., 1985⁵; Morris & Nicholson, 1987⁶).

In addition, there is now strong circumstantial evidence implicating *H*. *pylori* in the pathogenesis of gastric carcinoma (e.g. Jiang et al., 1987⁷; Lambert et al., 1986⁸; Crabtree et al., 1992⁹; 1993¹⁰; Forman et al., 1990¹¹, 1991¹²; Nomura et al., 1991¹³; Parsonnet et al., 1991¹⁴). Most recently, the Eurogast Study Group, led by Forman (1993¹⁵), demonstrated a significant relationship between *H*. *pylori* seropositivity and gastric cancer mortality and incidence. Indeed, there is now a convincing body of literature implying infection with *H*. *pylori* in a considerable proportion of upper gastrointestinal morbidity. A number of hypotheses have been suggested for the pathogenic mechanisms of *H*. *pylori* induced gastroduodenal disease, including the production of cytotoxins and mechanical disruption of the epithelium (e.g. Blaser, 1992¹⁶). Interestingly, however, many infected persons remain asymptomatic despite the persistent presence of the pathogen (Taylor & Blaser, 1991¹⁷).

J.Bacteriol. (1991), 173(2), 505-513 (O'Toole et al) describes a *Helicobacter pylori* protein.

J. Clin. Microbiol. (1991), Vol 29, 16-201624 (Drouet et al) describes another *Helicobacter pylori* protein.

### Statements of Invention

According to the invention, there is provided a vaccine for the treatment or prophylaxis of *Helicobacter pylori* infection or *Helicobacter pylori* associated disease, the vaccine comprising a *Helicobacter pylori* protein to which immunoreactivity is detected in *Helicobacter pylori* negative individuals, the protein including one or both of:
(a) a *Helicobacter pylori* protein having a molecular weight of 18 to 19 kDa and one or more of the following sequence characteristics: (i) an N-terminal amino acid sequence as listed in SEQ ID NO 2, or a portion thereof; (ii) an N-terminal amino acid sequence as listed in SEQ ID NO 6, or a portion thereof; (iii) an internal amino acid sequence as listed in SEQ ID NO 3, or a portion thereof; or a derivative, fragment or mutant of said *Helicobacter pylori* protein.
(b) a *Helicobacter pylori* protein having a molecular weight of 24 to 25 kDa and one or more of the following sequence characteristics: (i) an N-terminal amino acid sequence as listed in SEQ ID NO 1, or a portion thereof, (ii) an internal amino acid sequence as listed in SEQ ID NO 4, or a portion thereof; or a derivative, fragment or mutant of said *Helicobacter pylori* protein.

The vaccine may include a pharmaceutically acceptable carrier.

The vaccine may be combined with a suitable adjuvant such as interleukin 12 or a heat shock protein or both.

The vaccine may include at least one other pharmaceutical product such as an antibiotic and/or anti-bacterial agent such as bismuth salts. Typically the antibiotic is selected from one or more of metronidazole, amoxycillin, tetracycline, erythromycin, clarithromycin or tinidazole.

The vaccine is ideally for the treatment or prophylaxis of *Helicobacter pylori* infection or *Helicobacter pylori* associated disease(s).

The invention also provides the use of one or more *Helicobacter* proteins of the invention for the preparation of a medicament for the treatment or prophylaxis of *Helicobacter pylori* associated disease(s).

### Description of Drawings

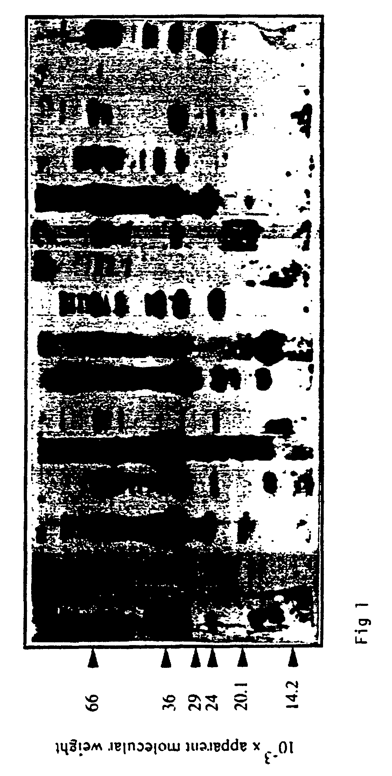

- Fig. 1:: Adult sera (CLO negative) screened for the presence of anti-*H*. *pylori* IgG antibodies. The figure shows a Western blot of *H. pylori* probed with serum obtained from CLO negative individuals. All sera were diluted 1:100 in PBS containing fat-free dried skimmed milk (5%, w/v). Proteins were transferred from SDS-PAGE gels to PVDF membrane. The antigen-antibody complexes were detected on washed membranes using an enhanced chemiluminescent detection system. Each track represents a different serum sample.

The invention also provides a method for the treatment or prophylaxis of *Helicobacter pylori* associated disease in a host, comprising administering to the host an immunologically effective amount of one or more of the *Helicobacter* proteins of the invention.

Preferably, the *Helicobacter pylori* protein is administered in combination with at least one other pharmaceutical agent.

In a preferred embodiment, the pharmaceutical agent is an antibiotic.

Ideally, the antibiotic is selected from one or more of metronidazole, amoxycillin, tetracycline or erythromycin, clarithromycin, tinidazole.

Typically the pharmaceutical agent includes an antibacterial agent such as bismuth salts.

In a preferred embodiment of the invention an adjuvant is administered in combination with the *Helicobacter* protein. Preferably the adjuvant is interleukin 12 or a heat shock protein or both.

The invention also provides the use of one or more *Helicobacter* proteins of the invention for the preparation of a medicament for the treatment or prophylaxis of *Helicobacter pylori* associated disease(s).

The invention further provides monoclonal or polyclonal antibodies or fragments thereof, to the proteinaceous material of the invention and purified antibodies or serum obtained by immunisation of an animal with the vaccine according to the invention.

The invention also provides the use of such serum and antibodies in the treatment or prophylaxis of *Helicobacter* associated disease(s) and in particular *Helicobacter pylori* associated disease(s).

The invention also provides a vaccine for the treatment or prophylaxis of *Helicobacter pylori* associated disease comprising an immunogenically effective amount of the *24 to 25 kDa Helicobacter pylori* protein and/or the 18 to 19 kDa *Helicobacter pylori* protein of the invention, an adjuvant such as Interleukin 12, and an antibiotic.

The vaccine may include an antibacterial agent such as bismuth salts.

The invention also includes the use of interleukin 12 in combination with the 18 to 19 kDa protein, the 24 to 25 kDa or any other *H. pylori* subunit as an adjuvant therapy.

Therefore, in another aspect, the invention provides a vaccine against *H. pylori* comprising an immunogenically effective amount of a *Helicobacter* or a subunit, fragment, derivative, precursor or mutant thereof in combination with interleukin 12 as an adjuvant. Preferably the *Helicobacter* is *Helicobacter pylori.*

In one embodiment of the invention the vaccine includes an antibiotic and may alternatively or additionally include an antibacterial agent.

### Description of Drawings

- Fig. 1 :: **Adult sera (CLO negative) screened for the presence of anti-*H*. *pylori* IgG antibodies**. The figure shows a Western blot of *H. pylori* probed with serum obtained from CLO negative individuals. All sera were diluted 1:100 in PBS containing fat-free dried skimmed milk (5%, w/v). Proteins were transferred from SDS-PAGE gels to PVDF membrane. The antigen-antibody complexes were detected on washed membranes using an enhanced chemiluminescent detection system. Each track represents a different serum sample.
- Fig. 2 :: **Absorbed sera :** Sera from two individuals negative for *H. pylori* were absorbed with either whole *C. jejuni* (track A), *H. pylori* (track B), or *E. coli* (track C).
- Fig. 3 :: **Partial purification of 18 and 25 kDa proteins :** Both proteins were purified from whole *Helicobacter pylori* on the basis of molecular weight using preparative continuous-elution SDS-PAGE on a Model 491 Prep-Cell (Bio-Rad).
- Fig. 4 :: **Sera obtained from CLO negative children screened for the presence of anti-*H*. *pylori* IgG antibodies.** The figure shows a Western blot of *H. pylori* probed with serum obtained from CLO negative children. All sera were diluted 1:50 in PBS containing fat-free dried skimmed milk (5%, w/v). Each track represents a different serum sample.
- Fig. 5 :: **Antigens recognised on *C. jejuni* and *E. coli* by anti-*H*. *pylori* antiserum**. The figure shows a Western blot of *H. pylori* (track A), *C*. *jejuni* (track B) and *E. coli* (track C) probed with rabbit anti-*H*. *pylori* antiserum. Each bacterium (5 µg) was subjected to SDS-PAGE followed by immunoblotting.
- Fig. 6 :: **Western blot of purified 25 kDa protein developed with serum from an individual negative for *H. pylori.*** Purified 25 kDa protein was subjected to SDS-PAGE and Western blotting. The blot was probed with serum obtained from a subject uninfected with *H*. *pylori.*

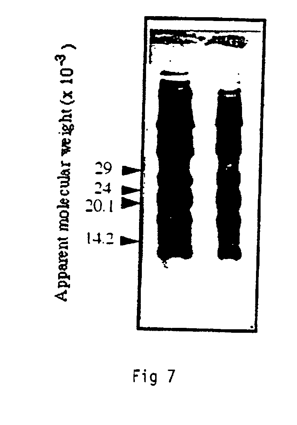

- Fig. 7 :: **Biotinylation of proteins located on the surface of *Helicobacter pylori***. Agar-grown *H*. *pylori* were harvested in phosphate buffered saline (pH 7.3) and washed twice in this buffer prior to biotinylation of surface exposed proteins. Bacteria (^{~}2 mg ml⁻¹) were resuspended in PBS (1 ml) and prewarmed to 37°C. Thereafter, biotin-X-NHS (Sulfosuccinimidyl-6(biotinamido)-hexanoate; Calbiochem) was added to a final concentration of 1 mM and was prepared immediately before use. After mixing to 10 min at 37°C, the labelling reaction was terminated by the addition of 1.5 M Tris-Cl (pH 8) to a final concentration of 10 mM. The suspension was washed three times by centrifugation (10,000 *g*, 1 min) in ice-cold PBS. Examination of the bacteria by light microscopy after the labelling and washing procedures demonstrated that the cells were still intact and motile. Biotinylated *H. pylori* was subjected to analytical SDS-PAGE, followed by Western blotting, to identify the biotinylated proteins. The Western blots were developed with Extravidin-peroxidase (Sigma).
- Fig. 8 :: Illustrates thymidine incorporation of lymphocytes in response to *H. pylori* in the presence and absence of interleukin 12.
- Fig. 9 :: Illustrates thymidine incorporation of peripheral blood mononuclear cells in the presence or absence of *H. pylori* with or without anti-interleukin 10 or recombinant interleukin 12.

### Detailed Description of the Invention

We have studied the prevalence of immuno-reactivity to *H. pylori* in both infected and un-infected individuals and found that un-infected individuals have a high response to *H. pylori* both in their B-cell and T-cell systems. Specifically, the T-cell immune response to *H. pylori* seems to be stronger in individuals who are negative for the organism. In this regard we have examined the secretion of the cytokine -interferon which is extremely important for the killing of microorganism by macrophages. Secretion of -interferon by T-cells of patients infected with *H. pylori* was considerably less than secretion by un-infected individuals when their T-cells were exposed to the organism (Fan et al., 1993¹⁸). Hence, these data suggest that individuals who are *H. pylori* negative have been exposed to the organism and may potentially have cleared the organism. Furthermore, the response to the organism is considerably more potent in this group of individuals than it is in the *H. pylori* positive patients.

The term *"H. pylori* negative individuals" means individuals with immunoreactivity to *H. pylori* who do not have evidence of *H. pylori* gastric colonisation as determined by techniques such as one or more of rapid urease testing, histological examination or culture of gastric biopsies.

A second component relates to the antibody response to *H. pylori* in *H. pylori* negative individuals. Briefly, we have demonstrated using a sensitive detection system that the majority of *H. pylori* negative individuals have detectable antibodies to two *H*. *pylori* proteins. Specifically, these proteins are of MW 18 - 19 and 24 - 25 kDa. It is thus proposed that a potent immune response to these antigens results in protective immunity to the organism. Furthermore, we have partially sequenced these proteins.

In many cases antibodies to *H. pylori* are detected by ELISA.

An inherent constraint in the design of ELISA based detection systems is that of establishing a cut off point such that all samples below this threshold are considered negative. Clearly, many seropositive cases will remain undetected in this situation and a true estimate of the incident of prior contact with the organism will thereby be underestimated. In this approach we use Western blotting to investigate antigen specificity of systemic responses to *H*. *pylori* in both healthy and *H. pylori*-infected individuals and shown that the incidence of seropositivity in *H*. *pylori* negative individuals is much greater than has previously been demonstrated. Furthermore, we have demonstrated that antibodies to a 24 to 25 kDa protein are detectable in the majority of *H. pylori* negative individuals. These were detected using a technique which we have modified called Enhanced Chemiluminescence. Enhanced Chemiluminescence on Western blot analysis reveals that the majority of uninfected individuals have antibodies which are specific for *H. pylori* and recognise antigens which are not present on other micro organisms. Of these antigens the most common one recognised is a 24 to 25 kDa protein which appears to be specific to *H. pylori.* Hence, these data suggest that immunisation with the 24 to 25 kDa protein or sub-unit thereof could have the potential to confer protective immunity on individuals who are either un-infected with the organism or individuals in whom the organism has been cleared by anti-bacterial treatment. A second protein was also identified at 18 to 19 kDa in a large subgroup of *H. pylori* negative individuals. Similarly, immunization with this protein or subunit thereof could also confer protective immunity.

We have developed a novel assay for detection of antibodies to *H. pylori.* This assay uses Western blotting and Enhanced Chemiluminescence (ECL). Using this assay we have demonstrated that approximately 75% of individuals who are negative for *H. pylori* by routine testing such as the rapid urease test have in fact got detectable antibodies to *H. pylori* (Fig. 1).

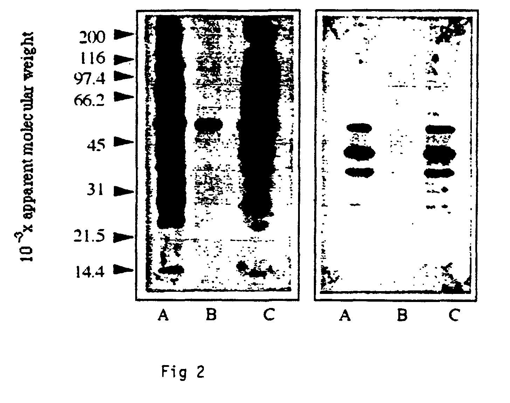

Furthermore, these antibodies are not absorbed by *C. jejuni* or by *E. coli* suggesting that this is a specific antibody response (Fig.2 ). Of particular note we have performed characterisation of the antigens recognised by these antibodies by molecular weight, using ECL Western blotting. Sera from un-infected individuals recognize a range of antigens on *H. pylori.* The most common antigen recognised is a 24 to 25 kDa protein which is recognised in over 70% of individuals who are negative for the organism on Rapid urease testing. Hence this suggests that the 24 to 25 kDa protein may be an immunodominant antigen which evokes a powerful immune response in individuals who are negative for the organism. A second protein was identified at 18 to 19 kDa which elicited significant antibody responses in *H. pylori*-negative children. These proteins have been further characterised by N-terminal and internal sequencing as outlined in the Appendix.

Finally a cytokine produced by macrophages called interleukin 12 may significantly enhance γ-interferon production in response to antigen. As stated previously, antigen-specific interferon production is reduced with *H. pylori* positive individuals. The addition of IL-12 to immunisation schedules with a 25 kDa protein would be expected to boost host immunity to *H*. *pylori* by augmenting the γ-interferon response.

Materials. All antibodies were obtained from Dako Ltd., High Wycombe, Bucks., U.K. All other chemicals and solvents were obtained from either the Sigma Chemical Company Ltd., Poole, Dorset, United Kingdom or BDH Chemicals Ltd., Poole, Dorset, United Kingdom.

SDS-PAGE. Discontinuous SDS-PAGE was performed essentially as described by Laemmli (1970)¹⁹. A total of 5 mg of acetone-precipitated *H. pylori* protein were located into each well. Gels were either stained with Coomassie Blue R-250 or processed for immunoblotting. Broad range molecular weight markers were purchased from Bio-Rad Laboratories, 3300 Regatta Blvd., Richmond, CA 94804. The molecular masses are expressed as kDa.

Western Blotting. Proteins from SDS-PAGE gels (30% T/2.67% C) were electroblotted (0.8 mA/cm² for 1 h) to PVDF membrane using a semi-dry blotting apparatus (LKB/Pharmacia), essentially as described by Towbin et al, (1979). Primary antibodies (human serum; 1/50 - 1/100 dilution) were detected using a 1/5,000 dilution of anti-human IgG (horseradish peroxidase-conjugated) in combination with enhanced chemiluminescence. Blots were washed in PBS containing fat-free dried skimmed milk (5%, w/v) and Tween-20 (0.05%, v/v). Blots were exposed to Kodak X-OMAT S film for 1-10 s. Exposed films were developed in Kodak LX-24 developer and fixed in Kodak dental X-ray fixer.

Sera. Serum samples were obtained from the Research Centre, Our Ladies Hospital for Sick Children, Crumlin, Dublin. All subjects were attended for medical conditions other than gastroenterological disorders. In addition, blood samples were obtained from a randomly selected cohort of children (Harcourt Street Childrens Hospital, Dublin) or from adults attending the gastenterology unit at St. James's Hospital, Dublin. All patients had a rapid urease (CLOtest) performed. Patients were defined as *H. pylori* positive or negative on the basis of positive or negative responses on rapid urease test.

Anti-*H. pylori* antiserum. Anti-*H*. *pylori* antiserum was a kind gift from Prof. B. Drumm and Dr. M. Clyne. The antiserum was raised in New Zealand white rabbits against whole *H. pylori* using conventional immunizing and boosting procedures.

Protein Measurements. Protein was measured by the method of Markwell et al. (1978)²⁰ with bovine serum albumin as the protein standard.

Absorption of sera. Antisera were absorbed with either *E. coli or C*. *jejuni* by incubating a suspension of the bacteria with patient sera for 2 h at room temperature with gentle mixing. The bacteria were removed from suspension by centrifugation (12,000 x g, 3 min).

Bacterial strains and growth conditions. The clinical isolates *H*. *pylori* used in this study were isolated from antral biopsies obtained from patients attending the gastroenterology clinic at St. James's Hospital, Dublin. *H. pylori* was grown under microaerophilic conditions for 4 days on 7% lysed horse blood agar at 37°C. Cells were harvested into ice-cold phosphate buffered saline (pH 7.5) containing PMSF (1 mM), EDTA (1 mM), and leupeptin (50 µg/ml). The cells were washed twice by centrifugation (10,000 x g, 5 min, 4°C) in this buffer before use. *C. jejuni* was a clinical isolate from stool in a patient with *C*. *jejuni* enteritis and was grown for two days exactly as described above with the exception that the incubation temperature was 42°C. The strain of *E. coli* used in this study is commercially available (Gibco) NTCC 11637 and was kindly provided by Dr. Ciaran Cronin, Dpt. Pharmacology, University College Dublin.

### Methods used in the identification and partial purification of two novel antigens from Helicobacter pylori

### Methods

Western Blotting. Proteins from SDs-PAGE gels (30% T/2.67% C) were electroblotted (0.8 mA/cm² for 1 h) to PVDF membrane using a semi-dry blotting apparatus (LKB/Pharmacia). Primary antibodies (human serum; 1/50 - 1/100 dilution) were detected using a 1/5,000 dilution of anti-human IgG (horseradish peroxidase-conjugated) in combination with enhanced chemiluminescence (see below). Blots were washed in phosphate buffered saline (pH 7.5) containing fat-free dried skimmed milk (5%, w/v) and Tween-20 (0.05%, v/v). Blots were exposed to Kodak X-OMAT S film for 1-10 s. Exposed films were developed in Kodak LX-24 developer and fixed in Kodak dental X-ray fixer.

### Enhanced Chemiluminescence (ECL)

The use of chemiluminescence to detect antibodies in Western blotting in preference to the conventional procedures of employing chromogenic substrates as detection reagents was adopted primarily because of the reporting gain in the sensitivity of detection (approximately 10-fold) over that found when chromogens are used. Oxidized luminol emits visible light and the intensity of this light emission is increased 1000-fold in the presence of chemical enhancers (e.g. iodophenol). The method is described blow:

| **Substrate** | **Concentration/Amount** |
|---|---|
| Luminol | 1.2 mM (in 0.1 M-Tris (50ml), pH 8.8) |
| 4-Iodophenol | 0.4 mM (dissolved in DMSO before use) |
| Hydrogen Peroxide | 17 µl of a 30% (v/v) solution |

Blots were incubated in the above mixture for one minute and then exposed to X-ray film as described above.

### Partial Purification of 18 and 25 kDa Proteins

Both proteins were partially purified from whole *Helicobacter pylori* on the basis of molecular weight (Fig. 2) using preparative continuous-elution sodium dodecyl sulphate polyacrylamide gel electrophoresis (SDS-PAGE) on a Model 491 Prep-Cell (Bio-Rad). This method enables us to quantitatively purify preparative amounts of proteins in a soluble form.

### Purification Method

25 mg *H. pylori* were precipitated with ice-cold acetone, washed once in acetone and the precipitate then solubilised in 3.8 ml SDS-PAGE sample buffer (62 mM Tris, pH 6.8; glycerol (10%, v/v); SDS (2%, v/v); 2-mercaptoethanol (5%, v/v); bromophenol blue (0.002%, v/v). Published electrophoretic procedures, with very minor modifications, were followed throughout sample preparation.

Loading: The protein mixture, in sample buffer, was loaded onto a 12.5% polyacrylamide tube gel (30% T/2.67% C). The dimensions of the tube gel were: 28 mm internal diameter; upper surface 3.6 cm²; stacking gel 2 cm; resolving gel 10 cm.

Running Conditions: Electrophoresis was performed at 40 mA (constant current) overnight at room temperature. Fractions (1 ml) were collected at 0.1 ml/min. Samples of each fraction (5 µl) were subjected to analytical SDS-PAGE to assess the purity and antigenicity of each protein. Every fraction within the molecular mass region of interest was screened by both SDS-PAGE (to assess purity) and Western blotting (to assess antigenicity) in an attempt to isolate and characterise the individual immunogenic proteins. The resolution of this technique is such that pure preparations of single proteins may be achieved once optimal electrophoretic conditions have been established. Preliminary optimization protocols entailed electrophoresing mixtures of *H. pylori* proteins under conditions designed to favour high resolution of low molecular weight proteins. The final electrophoretic conditions used to achieve partial purification of the selected proteins are detailed in the Methods section. Using these exact conditions the 18 kDa proteins eluted between 11-14 ml and the 25 kDa protein eluted within 16-20 ml. The molecular weights of the proteins were determined by analytical SDS-PAGE using a range of low molecular weight marker proteins (range: 14.5 kDa - 66 kDa; code: Sigma SDS-7) and Western blotting confirmed that these proteins were the immunogens of interest.

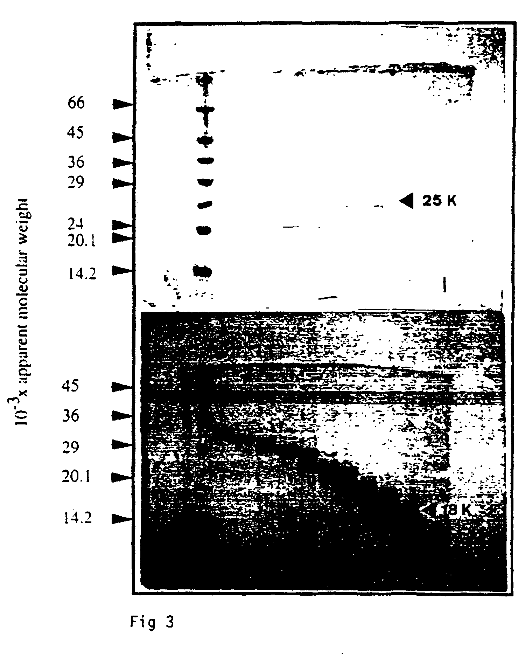

Figure 1 shows Western blot analysis of antibody responses to *H. pylori* in individuals negative for *H. pylori* on Rapid urease testing. Western blotting was performed as previously described using an enhanced chemiluminescence detection system. Antibodies to a large range of *H*. *pylori* proteins were seen in individuals who are *H*. *pylori* negative on Rapid urease testing. The most common antigen to which an antibody was detected with the 25 kDa protein. Figure 3 shows a preparative SDS gel elution profile of the 25 kDa and 18 kDa proteins. These proteins have been further characterised by N-terminal and internal sequencing as outlined in the Appendix.

### EXAMPLE 1

### CLO negative adults

Similarly, a cohort of 19 adult sera was screened for anti-*H. pylori* IgG antibodies. Each of these subjects was CLO negative, yet 83% had detectable antibodies (IgG) to *H. pylori* (Fig. 1). Taken together, these data suggest extensive prior contact with *H. pylori.* The most common antigen to which an antibody was detected was a 25 kDa species.

### CLO negative children

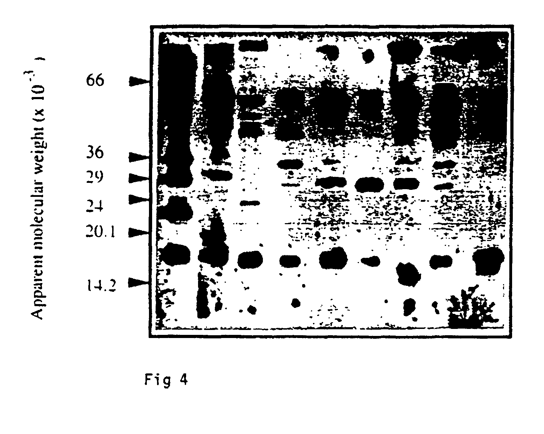

The systemic humoral immune response (IgG) to *H. pylori* was studied in two groups of children also. None of these subjects had received any form of anti-*H*. *pylori* therapy. However, in almost all cases the children had a specific antibody response to *H. pylori.* The first cohort studies consisted of twenty children (age range: 4 - 15 years), negative for *H*. *pylori* on CLO test. Of these, 75% had detectable IgG antibodies to *H. pylori* (Fig. 4).

The second cohort of children (n = 20) were asymptomatic and presented in hospital with conditions other than gastrointestinal disorders. Yet 13/18 (72%) had detectable IgG antibodies to several *H*. *pylori* specific antigens. However, from the intensity of the response the data suggest that the antibody response is most likely due to prior contact with the bacterium, when compared to the considerably stronger response observed with *H. pylori* positive individuals.

### EXAMPLE 2

### Cross Reactivity with other Bacteria

As many bacteria share common antigenic determinants, we examined the extent of cross-reactivity between *H. pylori* and the closely related *C*. *jejuni,* in addition to *E. coil*, using two complimentary approaches. Firstly, the ability of the anti-*H*. *pylori* polyclonal antiserum to recognise antigens on both *C. jejuni* and *E. coli* was examined by Western blotting (Fig. 2).

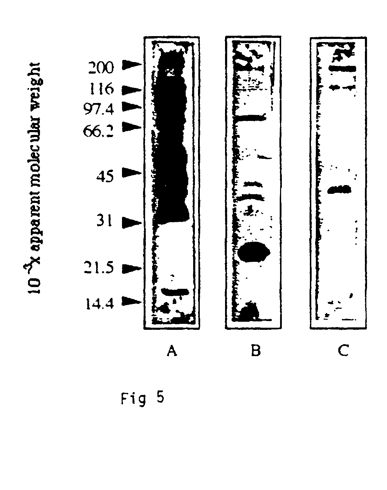

Anti-*H*. *pylori* antiserum recognized a number of antigenic determinants on both *E*. *coli* and *C. jejuni*. Specifically, the antiserum recognises proteins of molecular mass 72, 50, 40, 36, and 25 kDa on *C. jejuni* and proteins of molecular mass 200, 116, 45, and 38 kDa on *E*. *coli* (Fig. 5). Of these, only 3 proteins (70, 25 kDa from *C*. *jejuni* and 200 kDa from *E. coli*) show pronounced cross-reactivity with anti-*H*. *pylori* antiserum. Therefore, the observed cross reactivity is clearly not extensive. Secondly, absorption experiments demonstrated that this cross reactive antigen recognition was of minor significance. Serum samples absorbed with clinical isolates of *H. pylori* and *C. jejuni* in addition to a commercially available strain of *E. coli* demonstrated that seroreactivity could be eliminated by absorbing with *H*. *pylori* but not with *C. jejuni* or *E*. *coli* (Fig. 2). Figure 2 is a representative experiment. Absorption studies were performed on approximately half of the serum samples screened in this study with similar results to those shown. The 18 and 25 kDa proteins were also detected in *H. pylori* Reference Strains NTCC 11637 and 11638 in addition to all clinical strains tested.

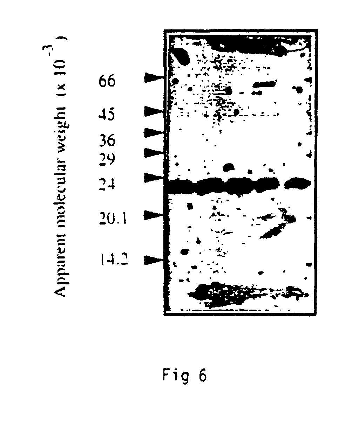

Having partially purified the 26-26 kDa protein by preparation continuous-elution electrophoresis as shown in Fig. 3, we confirmed the antigenicity of the 24-26 kDa protein by probing a Western blot of purified 24-26 kDa protein with serum from an uninfected individual (Fig. 6). The example shown in Fig. 6 is a representative experiment where the blot was incubated with the serum from an *H. pylori* un-infected individual. Clearly, this serum sample contains antibodies that specifically recognise the 24-26 kDa protein and furthermore, the results of this experiment demonstrate that the antigen preparation is highly enriched for this protein and that no other immunogenic proteins are present in this preparation. We have obtained similar results with the 18-20 kDa protein.

### Example 3

### Biotinylation of whole intact Helicobacter pylori

Agar-grown *H. pylori* were harvested in phosphate buffered saline (pH 7.3) and washed twice in this buffer prior to biotinylation of surface exposed proteins. Bacteria (^{~}2 mg ml⁻¹) were resuspended in PBS (1 ml) and prewarmed to 37°C. Thereafter, biotin-X-NHS (Sulfosuccinimidyl-6(biotinamido)-hexanoate; Calbiochem) was added to a final concentration of 1mM and was prepared immediately before use. After mixing for 10 minutes at 37°C, the labelling reaction was terminated by the addition of 1.5 M Tris-Cl (pH 8) to a final concentration of 10 mM. The suspension was washed three times by centrifugation (10,000 g, 1 min) in ice-cold PBS. Examination of the bacteria by light microscopy after the labelling and washing procedures demonstrated that the cells were still intact and motile.

### Analysis of biotinylated proteins

Biotinylated *H*. *pylori* was subjected to both analytical and preparative SDS-PAGE, followed by Western blotting, to identify the biotinylated proteins. The Western blots were developed with Extravidin-peroxidase (Sigma). Extensive incorporation of the biotin ester into *H. pylori* proteins was observed (Fig. 7). Furthermore, it is clear from this figure that proteins in the 18-24 kDa region are biotinylated as are a number of other proteins (Table 1), indicating that these proteins are present on the surface of the bacterium.

**Table 1**

| **Biotinylated Protein** | **Apparent molecular weight** |
|---|---|
| 1 | 13,800 |
| 2 | 15,600 |
| 3 | 16,600 |
| 4* | 17,700 |
| 5 | 20,500 |
| 6* | 23,500 |
| 7 | 26,400 |

### Method description

### T-cell immune response to Helicobacter pylori

We examined the T-lymphocyte proliferative responses to H. pylori using a thymidine incorporation assay. Briefly, lymphocytes were isolated by density gradient centrifugation on a Ficoll-Hypaque gradient. Lymphocytes were seeded into 96-well microtitre plates at a density of 10⁵ cells/well in RPMI 1640 medium containing 10% foetal calf serum. A sonicated irradiated preparation of H. pylori was added at a concentration of 3µg/ml. Medium alone was added to control wells. In addition interleukin 12 (R&D suppliers) was added at a concentration of 500 pg/ml. Cells were then cultured in a 5% CO₂ incubator for 4 days at 37°C. At 4 days tritiated thymidine 1 µCi/ml was added and cultures continued for a further 24 hours before harvesting using a multiple automated sample harvester. In additional experiments, cells were stimulated using OKT3 antibody to the CD3 T-cell receptor associated complex in the presence and absence of the H. pylori preparation as above. In these studies, interleukin 12 was similarly added. Antibody to interleukin 10 was added in some experiments.

### Example 4

### T-cell response to H. pylori is significantly augmented by interleukin 12

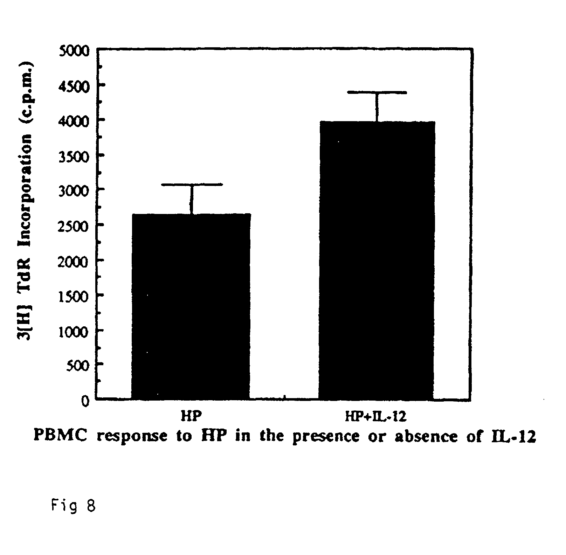

In a cohort of patients in whom lymphocyte proliferative responses to H. pylori were examined as described in the methodology, interleukin 12 significantly increased the proliferation of the peripheral blood mononuclear cell population to H. pylori (n=12, p<.05) (Fig. 8). These data demonstrate clearly that interleukin 12 has adjuvant properties in respect of H. pylori immunogenicity.

### Interleukin 12 overcomes the suppression of T-cell responses induced by H pylori

The H pylori antigen preparation significantly inhibited the proliferation induced by the T-cell mitogen OKT3. This inhibition could be abolished using antibody to interleukin 10, a cytokine produced by T-helper 2 cells known to suppress the T-helper 1 cell pathways involved in cell proliferation. These data therefore suggest that the suppression of T-cell proliferation induced by H pylori is mediated by interleukin 10 through a T-helper 2 pathway. Interleukin 12 also abolished the suppression of T-cell responses induced by H pylori and significantly increased proliferative responses over the baseline OKT3-induced response suggesting that this cytokine is capable of overcoming the effects of the H. pylori T-helper 2 pathway.

Fig. 8 illustrates thymidine incorporation of lymphocytes in response to H. pylori in the presence and absence of interleukin 12. Interleukin 12 significantly augmented proliferation of peripheral blood mononuclear cells in response to H. pylori.

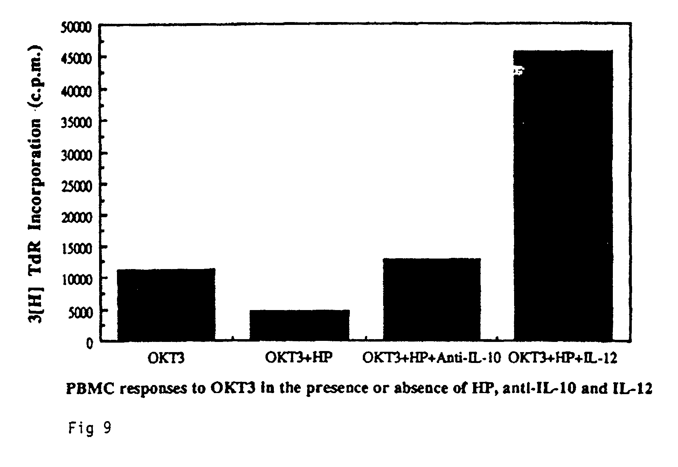

Fig. 9 illustrates thymidine incorporation of peripheral blood mononuclear cells in the presence or absence of H pylori with or without anti-interleukin 10 or recombinant interleukin 12. Both interleukin 12 and anti-interleukin 10 significantly abolished H pylori-induced inhibition of lymphocyte proliferation.

It will be appreciated that interleukin 12 may also be used as an adjuvant with any *H. pylori* protein or derivative or fragment thereof. Its application is not limited to the specific 25 kDa or 18 kDa proteins referred to above. The interleukin 12 may be conjugated with the H. pylori unit in such a way as to allow the interleukin to be released in vivo, for example by peptic acid and gastric enzymes/or urease.

It will be appreciated by those skilled in the art that while we have referred to a molecular mass of 24 to 25 kDa and 18 to 19 kDa the molecular mass may lie in the 24-26 kDa and 17-19 kDa range. Other related organisms such as H. Felis or H. mustelis may produce gastric diseases in animal models.

Cross reactivity between proteins from *Helicobacter* species may mean that antigens from an individual bacterial species could provide protection in an animal which is not its normal host.

The dominant antigens to which antibody is detected in *Helicobacter pylori*-negative individuals are the 18-19 and 24-25 kDa antigens. Hence, use of an antigenic preparation containing all antigens less than 30 kDa, preferably less than 29, ideally less than 28 and preferably less than 27 kDa and would be enriched in the immunodominant antigens to be used in putative vaccine.

It will be apparent that cytokine interleukin 12 acts as an adjuvant to potentiate the immunogenicity of *H. pylori.* In particular, it potentiates the immunogenicity of protein fractions of less than 30 kDa, especially the 18 kDa and 25 kDa protein fractions of *H*. *pylori.*

It will be appreciated that interleukin 12 may also be used as an adjuvant with any *H. pylori* protein or derivative or fragment thereof. Its application is not limited to the specific 25 kDa or 18 kDa proteins referred to above. The interleukin 12 may be conjugated with the H. pylori unit in such a way as to allow the interleukin to be released in vivo, for example by peptic acid and gastric enzymes/or urease.

It will be appreciated by those skilled in the art that while we have referred to a molecular mass of 24 to 25 kDa and 18 to 19 kDa the molecular mass may lie in the 24-26 kDa and 17-19 kDa range. Other related organisms such as H. Felis or H. mustelis may produce gastric diseases in animal models.

Cross reactivity between proteins from *Helicobacter* species may mean that antigens from an individual bacterial species could provide protection in an animal which is not its normal host.

The dominant antigens to which antibody is detected in *Helicobacter pylori*-negative individuals are the 18-19 and 24-25 kDa antigens. Hence, use of an antigenic preparation containing all antigens less than 30 kDa, preferably less than 29, ideally less than 28 and preferably less than 27 kDa and would be enriched in the immunodominant antigens to be used in putative vaccine.

### Partial sequencing of the two antigens from Helicobacter pylori

### N-terminal sequence analysis

Purified 18 and 24 kDa proteins were electroblotted to PVDF and ProBlott, respectively, from 12.5% polyacrylamide gels. The proteins were located on the membranes by staining with 0.1% amido black (in 1% acetic acid, 40% method) for 15s followed by destaining in several changes of distilled deionized water. The membranes were air-dried thoroughly and submitted for sequence analysis using the Edman degradation procedure as described by Matsudaira (1989²¹).

The N-terminal amino acid sequence of the 25 and 18 kDa protein are given in Sequence Id No's 1 and 2 respectively.

### Peptide Mapping

The N-chlorosuccinimide peptide mapping method of Lischwe and Ochs (1982)²² was used with minor modifications. Bands of interest were located on SDS-PAGE gels (12.5% T) by briefly staining the gel with 0.1% Coomassie Blue R250 (in 50% methanol, 10% acetic acid) and then excised with a scalpel blade. The protein present in the gel slices was digested with N-chlorosuccinimide (15 mM) in acetic acid/urea/water (1:1:1, v/w/v) for 30 min at 20°C. The treated gel slices were then washed with several changes of water and equilibrated with SDS-PAGE sample buffer exactly as described by Lischwe and Ochs. Finally, the gel slices were placed in the sample wells of a 15% polyacrylamide SDS-PAGE gel and electrophoresed. Following electrophoresis, the separated peptides were transferred to either PVDF or ProBlott by Western blotting. Peptides were visualized by staining the membrane with 0.1% amido black in acetic acid (1%) and methanol (40%). After extensive washing with water, the peptides were submitted for sequencing without any further modifications.

Mercaptoacetic acid (2 mM) was included in the upper electrode buffer during all SDS-PAGE electrophoretic procedures. This mobile thiol behaves as a free radical scavenger and thus prevents N-blocking.

Amino acid sequences for internal peptides from the 18 and 25 kDa protein are given in Sequence Id. No.'s 3 and 4 respectively.

### Extraction of Helicobacter pylori chromosomal DNA

Chromosomal DNA was extracted as described (Silhavy et al., 1984. Experiments with gene fusions. C.S.H. publications).

Amplifying the sequence of the 18-19 protein kDa gene of using degenerate primers.

Degenerate DNA sequence was deduced from the amino acid sequences listed in Sequence Id. No.'s 2 and 3. Four degenerate primers were designed from these sequences, to allow for a two stage, nested, PCR reaction. *Eagl* restriction enzyme sites were built into each primer, to allow for subsequent cloning of the fragment. Where three or more bases were possible at any site, inosine was incorporated instead of all possible bases, except, where such sites were four bases or less from the primers 3' (3 prime) terminal, in which case all possible bases were included. Inosine was also avoided at positions immediately adjacent to the *Eagl* sites.

### Degenerate primers for gene p18:

1. GAARA CGGCC GARAT IYTIA ARCAY YTICA RGC

2. TCYTC GGCCG TYTCY TCIGT NGCY

3. RATIY TCGGC CGYYI CARGC IGAYG C

4. ATYTC GGCCG TIGCY TTRTG NAC

Genomic DNA for the 18 - 19 kDa protein gene p18 was amplified as follows using the outer set of primers (primers 1 & 2): the samples were heated to 94 degrees C for 3 minutes to denature the DNA, followed by 35 cycles of 94 degrees C for 30 seconds, 56 degrees C for 40 seconds and 72 degrees C for 30 seconds. 100 pmol of each primer was used, in the presence of 2.5 mM MgCl₂ and 0.2 mM dNTPs, in a reaction volume of 50 ul. 1 ul of this reaction was used as the substrated for the 'nested' reaction. This reaction was the same as outlined for the above reaction, except that the inner primers (primers 3 & 4) were substituted for the external primers, and a concentration of 2.0 mM MgCl₂ was used. Electrophoresis of the products of the reaction resulted in a clearly visible band on a 2% agarose gel, estimated at approximately 120 bp in size (as judged by a molecular size ladder).

Sequencing the amplified DNA sequence.

The nested PCR fragment corresponding to the 18 - 19 kDa protein gene was cloned by digesting the fragment with *Eagl* and ligating this into the unique *Eagl* site in the Bluescript vector (Stratagene). E. coli cells were transformed (according to standard procedures) and plasmid DNA was harvested using the alkaline lysis method (Sambrook et al., 1989. Molecular cloning : A laboratory manual 2nd. Ed., CSH publications) followed by an RNAase digestion step, phenol/chloroform extraction and precipitation using 2.5M ammonium acetate and 2 volumes of ethanol. Two independent isolates of plasmid DNA were sequenced using forward and reverse universal sequencing primers. The inserted DNA derived from the p18 gene was sequenced in the forward and reverse orientations. Sequencing was performed using an ABI automated sequencer and a Genpak PCR based fluorescent dideoxy chain terminator termini sequencing kit.

The sequence of bases between the terminal of the internal PCR primers is :

GATCGTGTTATTTATGAAAGTGCATAACTTCCATTGGAATGTGAAAGGCAC
CGATTTTTTCAAT

This sequence of bases translates into the amino acid sequence listed in Sequence Id. No. 5.

This sequence (Sequence Id. No. 5) overlaps with both the 18 kDa protein N-terminal amino acid sequence listed in Sequence Id. No. 2 and the 18 kDa protein internal amino acid sequence listed in Sequence No. 3, to give the enlarged N-terminal amino acid sequence listed in Sequence Id. No. 6.

Many variations on the specific embodiments described will be readily apparent and accordingly the invention is not limited to the embodiments hereinbefore described which may be varied in detail.

### LIST OF REFERENCES

1. Marshall, B.J. and Warren, J.R. (1984). Unidentified curved bacilli in the stomach of patients with gastritis and peptic ulceration. *Lancet* **1**, 1311-1314.
2. Blaser M.J. (1990). *Helicobacter pylori* and the pathogenesis of gastrodudodenal inflammation. *J. Infect*. *Dis.* **161**, 626-633.
3. Rauws, E.A.J. and Tytgat, G. N. J. (1990). Eradication of *Helicobacter pylori* cures duodenal ulcer : *Lancet* **1**, 1233-1235.
4. Lambert, J.R., Borromeo, M., Pinkard, K.J., Turner, H., Chapman, C.B., and Smith, M.L. (1987). Colonisation of gnotobiotic pigs with Campylobacter pylori - an animal model ? *J. Infect. Dis.* **155,** 1344.
5. Marshall, B.J., Armstrong, J.A., McGechie, D.B., and Glancy, R.J. (1985). Attempt to fulfil Koch's postulates for pyloric Campylobacter. *Med. J*. *Aust.* **142,** 436-439.
6. Morris, A. and Nicholson, G. (1987). Ingestion of Campylobacter pylori causes gastritis and raises fasting gastric pH. *Am. J. Gastroenterol.* **82**, 192-199.
7. Jiang, S.J., Liu, W.Z., Zhang, D.Z., Shi, Y., Xiao, S.D., Zhang, Z.N., and Liu, D. Y. (1987). Campylobacter-like organisms in chronic gastritis, peptic ulcer and gastric carcinoma. *Scand. J*. *Gastroenterol.* **22,** 553-558.
8. Lambert, J. R., Dunn, K.A., Eaves, E.R., Korman, M.G., and Hansky, J. (1986). Campylobacter pyloridis in diseases of the human upper gastrointestinal tract. *Gastroenterology* **90,** 1509.
9. Crabtree, J.E., Figura, N., Taylor, J.D., Bugnoli, M., Armellini, D., and Tompkins, D. S. (1992). Expression of 120 kDa protein and cytotoxicity in *Helicobacter pylori. J. Clin*. *Pathol.* **45,** 733-734.
10. Crabtree, J.E., Wyatt, J.I., Sobala, G.M., Miller, G., Tompkins, D.S., Primrose, J.N., and Morgan, A.G. (1993). Systemic and mucosal humoral responses to *Helicobacter pylori* in gastric cancer. Gut **34,** 1339-1343.
11. Forman, D., Sitas, F.,. and Newell, D.G. (1990). Geographic association of *Helicobacter pylori* antibody prevalence and gastric cancer mortality in rural China. *Int*. *J. Cancer* **46**, 608-611.
12. Forman, D., Newell, D.G., Fullerton, F., Yarnell, J.W.G., Stacey, A.R., Wald, N., and Sitas, F. (1991). Association between infection with *Helicobacter pylori* and risk of gastric cancer : evidence from a prospective investigation. *BMJ* **302,** 1302-1305.
13. Nomura, A., Stemmermann, G.N., Chyou, P-H., Kato, I., Perez-Perez, G.I., and Blaser, M.J. (1991). *Hellcobacter pylori* infection and gastric carcinoma amongst Japanese Americans in Hawaii, *N. Engl. J. Med.* **325,** 1132-1136.
14. Parsonnet, J., Friedman, G.D, Vandersteen, D.P., Chang, Y., Vogelman, J.H., Orentreich, N., and Sibley, R.K. (1991). *Helicobacter pylori* infection and the risk of gastric carcinoma. N. *Engl. J. Med.* **325,** 1127-1131.
15. Forman, D. (1993). An international association between *Helicobacter pylori* infection and gastric cancer. The EUROGAST Study Group. *Lancet* **341,** 1359-1362.
16. Blaser, M.J. (1992). Hypothesis on the pathogenesis and natural history of *Helicobacter pylori*-induced inflammation. *Gastroenterology* **102,** 720-727.
17. Taylor, D.N. and Blaser, M.J. (1991). Epidemiology of *Helicobacter pylori* infection. *Epidemiol. Rev.* **13,** 42-59.
18. Fan, X.J., Chua, A. Shahi, C.N., McDevitt, J., Keeling, P.W.N. and Kelleher, D. (1994) Gastric T lymphocyte responses to *Helicobacter pylori* colonisation. *Gut* **35**, 1379-1384.
19. Laemmli, U.K. (1970). Nature 227, 680-685.
20. Markwell, M.A.K., Haas, S.M., Bieber, L.L. and Tolbert, N.E. (1978) *Analytical Biochemistry,* **87**, 206-210.
21. Matsudaira, P.T. (1989). A practical guide to protein and peptide purification for microsequencing. Academic Press, San Diego.
22. Lischwe, M.A. and Ochs, D. (1982). A new method for partial peptide mapping using N-chlorosuccinimide/urea and peptide silver staining in sodium dodecyl sulfatepolyacrylamide gels. *Analytical Biochemistry* **127**, 453-457.

### APPENDIX

### SEQUENCE LISTING

(1) GENERAL INFORMATION
   (I) APPLICANT
      (A) NAME : RICAN LIMITED
      (B) STREET : 1 STOKES PLACE,
      (C) CITY : DUBLIN 2,
      (D) COUNTRY :IRELAND
      (E) POSTAL CODE :
      (F) TELEPHONE :353-1-2881230
      (G) TELEFAX : 353-1-2883439
   (II) TITLE OF INVENTION : *Helicobacter Proteins and Vaccines*
   (III) NUMBER OF SEQUENCES : 6
   (IV)
   (V) CURRENT APPLICATION DATA :
      APPLICATION NO. :
(2) INFORMATION FOR SEQUENCE ID. NO. : 1
   (I) SEQUENCE CHARACTERISTICS
      (A) LENGTH : 20 AMINO ACIDS
      (B) TYPE : AMINO ACID
      (C) TOPOLOGY : LINEAR
   (II) MOLECULE TYPE : PROTEIN
   (IV) ORIGINAL SOURCE :
      (A) ORGANISM : HELICOBACTER PYLORI
   (XI) SEQUENCE DESCRIPTION : SEO. ID. NO. 1 
Met-Leu-Val-Thr-Lys-Leu-Ala-Pro-Asp-Phe-Lys-Ala-Pro-Ala-
                5                   10
Val-Leu-Gly-Asn-Asn-Glu
15
(3) INFORMATION FOR SEQUENCE ID. NO. 2. :
   (I) SEQUENCE CHARACTERISTICS
      (A) LENGTH : 20 AMINO ACIDS
      (B) TYPE : AMINO ACID
      (C) TOPOLOGY : LINEAR
   (II) MOLECULE TYPE : PROTEIN
   (IV) ORIGINAL SOURCE :
      (A) ORGANISM : HELICOBACTER PYLORI
   (XI) SEQUENCE DESCRIPTION : SEO. ID. NO. 2 
Met-Lys-Thr-Phe-Glu-Ile-Leu-Lys-His-Leu-Gln-Ala-Asp-Ala-
5 10
Ile-Val-Leu-Phe-Met-Lys
15
 **NH2**
(4) INFORMATION FOR SEQUENCE ID. NO. 3 :
   (I) SEQUENCE CHARACTERISTICS
      (A) LENGTH : 20 AMINO ACIDS
      (B) TYPE : AMINO ACID
      (C) TOPOLOGY : LINEAR
   (II) MOLECULE TYPE : PROTEIN
   (IV) ORIGINAL SOURCE :
      (A) ORGANISM : HELICOBACTER PYLORI
   (XI) SEQUENCE DESCRIPTION : SEO. ID. NO. 3 
Asn-Val-Lys-Gly-Thr-Asp-Phe-Phe-Asn-Val-His-Lys-Ala-Thr-
5 10
Glu-Glu-Ile-Tyr-Glu-Glu
15 20
(5) INFORMATION FOR SEQUENCE ID. NO. : 4
   (I) SEQUENCE CHARACTERISTICS
      (A) LENGTH : 4 AMINO ACIDS
      (B) TYPE : AMINO ACID
      (C) TOPOLOGY : LINEAR
   (II) MOLECULE TYPE : PROTEIN
   (IV) ORIGINAL SOURCE :
      (A) ORGANISM : HELICOBACTER PYLORI
   (XI) SEQUENCE DESCRIPTION : SEO. ID. NO. 4 Lys-Asp-Thr-Pro
(6) INFORMATION FOR SEQUENCE ID. NO. 5:
   (I) SEQUENCE CHARACTERISTICS
      (A) LENGTH - 21 AMINO ACIDS
      (B) TYPE : AMINO ACID
      (C) TOPOLOGY : LINEAR
   (II) MOLECULE TYPE : PROTEIN
   (IV) ORIGINAL SOURCE :
      (A) ORGANISM : HELICOBACTER PYLORI
   (XI) SEQUENCE DESCRIPTION : SEO. ID. NO. 5 
Ile-Val-Leu-Phe-Met-Lys-Val-His-Asn-Phe-His-Trp-Asn-Val-
                5                   10
Lys-Gly-Thr-Asp-Phe-Phe-Asn
15                  20
(7) INFORMATION FOR SEQUENCE ID. NO. 6
   (I) SEQUENCE CHARACTERISTICS
      (A) LENGTH : 46 AMINO ACIDS
      (B) TYPE : AMINO ACID
      (C) TOPOLOGY : LINEAR
   (II) MOLECULE TYPE : PROTEIN
   (IV) ORIGINAL SOURCE :
      (A) ORGANISM : HELICOBACTER PYLORI

Met-Lys-Thr-Phe-Glu-Ile-Leu-Lys-His-Leu-Gln-Ala-Asp-Ala-
5 10
Ile-Val-Leu-Phe-Met-Lys-Val-His-Asn-Phe-His-Trp-Asn-Val-
15 20 25
Lys-Gly-Thr-Asp-Phe-Phe-Asn-Val-His-Lys-Ala-Thr-Glu-Glu-
30 35 40
Ile-Tyr-Glu-Glu
45

## Claims

1. A vaccine for the treatment or prophylaxis of *Helicobacter pylori* infection or *Helicobacter pylori* associated disease, the vaccine comprising a *Helicobacter pylori* protein to which immunoreactivity is detected in *Helicobacter pylori* negative individuals, the protein including one or both of:
(a) a *Helicobacter pylori* protein having a molecular weight of 18 to 19 kDa and one or more of the following sequence characteristics: (i) an N-terminal amino acid sequence as listed in SEQ ID NO 2, or a portion thereof; (ii) an N-terminal amino acid sequence as listed in SEQ ID NO 6, or a portion thereof; (iii) an internal amino acid sequence as listed in SEQ ID NO 3, or a portion thereof; or a derivative, fragment or mutant of said *Helicobacter pylori* protein.
(b) a *Helicobacter pylori* protein having a molecular weight of 24 to 25 kDa and one or more of the following sequence characteristics: (i) an N-terminal amino acid sequence as listed in SEQ ID NO 1, or a portion thereof; (ii) an internal amino acid sequence as listed in SEQ ID NO 4, or a portion thereof; or a derivative, fragment or mutant of said *Helicobacter pylori* protein.

2. A vaccine as claimed in claim 1 wherein the immunoreactivity is antibody based.

3. A vaccine as claimed in any preceding claim including a pharmaceutically acceptable carrier.

4. A vaccine as claimed in any preceding claim in combination with a pharmacologically suitable adjuvant.

5. A vaccine as claimed in claim 4 wherein the adjuvant is interleukin 12.

6. A vaccine as claimed in claim 4 or 5 wherein the adjuvant is a heat shock protein.

7. A vaccine as claimed in any preceding claim including at least one other pharmaceutical product.

8. A vaccine as claimed in claim 7 wherein the pharmaceutical product is an antibiotic, preferably the antibiotic is selected from one or more of metronidazole, amoxycillin, tetracycline or erythromycin, clarithromycin or tinidazole.

9. A vaccine as claimed in claim 7 or 8 wherein the pharmaceutical product includes an antibacterial agent such as bismuth salts.

10. A vaccine as claimed in any preceding claim in a form for oral administration.

11. A vaccine as claimed in any of claims 1 to 9 in a form for intranasal administration.

12. A vaccine as claimed in any of claims 1 to 9 in a form for intravenous administration.

13. A vaccine as claimed in any of claims 1 to 9 in a form for intramuscular administration.

14. A vaccine as claimed in any of claims 1 to 9 including a peptide delivery system.

15. The use of *Helicobacter pylori* protein as defined in claim 1 or a derivative or fragment or mutant thereof for the preparation of a medicament for the treatment or prophylaxis of *Helicobacter pylori* associated disease(s).

16. The use as claimed in claim 15 wherein the medicament which is prepared is a vaccine as claimed in any of claims 2 to 14.

17. A method for preparing à vaccine as claimed in any of claims 1 to 14 which treats or prevents *Helicobacter pylori* infection or *Helicobacter pylori* associated disease, the method comprising:
obtaining a *Helicobacter pylori* protein as defined in claim 1; and
forming a vaccine preparation comprised of said protein or derivative or fragment or mutant thereof, which is suited for administration to a host and which when administered raises an immune response.

## Patentansprüche

1. Impfstoff zur Behandlung oder Vorbeugung einer *Helicobacter pylori*-Infektion oder einer *Helicobacter pylori*-assoziierten Krankheit, umfassend ein *Helicobacter pylori*-Protein, gegen welches Immunreaktivität in *Helicobacter pylori*-negativen Individuen festgestellt wird, wobei das Protein eine oder beide der folgenden Komponenten umfasst:
(a) ein *Helicobacter pylori*-Protein mit einem Molekulargewicht von 18 bis 19 kDa und einer oder mehrerer der folgenden Sequenzeigenschaften:
(i) eine N-terminale Aminosäuresequenz wie aufgeführt unter SEQ ID NO.: 2, oder ein Teil davon;
(ii) eine N-terminale Aminosäuresequenz wie aufgeführt in SEQ ID No.: 6, oder ein Teil davon;
(iii) eine interne Aminosäuresequenz wie aufgeführt in SEQ ID NO.: 3, oder ein Teil davon; oder ein Derivat, Fragment oder eine Mutante des *Helicobacter pylori*-Proteins,
(b) ein *Helicobacter pylori*-Protein mit einem Molekulargewicht von 24 bis 25 kDa und einer oder mehrerer der folgenden Sequenzeigenschaften:
(i) eine N-terminale Aminosäuresequenz wie aufgeführt unter SEQ ID NO.: 1, oder ein Teil davon;
(ii) eine interne Aminosäuresequenz wie aufgeführt in SEQ ID NO.: 4, oder ein Teil davon; oder ein Derivat, Fragment oder eine Mutante des *Helicobacter pylori*-Proteins.

2. Impfstoff nach Anspruch 1, wobei die Immunreaktivität auf Antikörpern basiert.

3. Impfstoff nach einem der vorhergehenden Ansprüche, einschließlich eines pharmazeutischen Trägers.

4. Impfstoff nach einem der vorhergehenden Ansprüche in Kombination mit einem pharmakologisch passenden Adjuvans.

5. Impfstoff nach Anspruch 4, wobei das Adjuvans Interleukin 12 ist.

6. Impfstoff nach Anspruch 4 oder 5, wobei das Adjuvans ein Hitzeschock-Protein ist.

7. Impfstoff nach einem der vorhergehenden Ansprüche, einschließlich mindestens eines anderen pharmazeutischen Produkts.

8. Impfstoff nach Anspruch 7, wobei das pharmazeutische Produkt ein Antibiotikum ist, vorzugsweise ausgewählt aus einem oder mehreren Mitgliedern der Gruppe bestehend aus Metronidazol, Amoxycillin, Tetracyclin, Erythromycin, Clarithromycin oder Tinidazol.

9. Impfstoff nach Anspruch 7 oder 8, wobei das pharmazeutische Produkt ein antibakterielles Agens wie zum Beispiel Wismutsalze einschließt.

10. Impfstoff nach einem der vorhergehenden Ansprüche, in einer Form zur oralen Verabreichung.

11. Impfstoff nach einem der Ansprüche 1 bis 9, in einer Form zur intranasalen Verabreichung.

12. Impfstoff nach einem der Ansprüche 1 bis 9, in einer Form zur intravenösen Verabreichung.

13. Impfstoff nach einem der Ansprüche 1 bis 9, in einer Form zur intramuskulären Verabreichung.

14. Impfstoff nach einem der Ansprüche 1 bis 9, einschließlich eines Peptid-Verabreichungssystems.

15. Verwendung des *Helicobacter pylori*-Proteins gemäß der Definition in Anspruch 1 oder eines Derivats oder Fragments oder einer Mutante davon, zur Herstellung eines Medikaments zur Behandlung oder Vorbeugung von *Helicobacter pylori*-assoziierten Krankheiten.

16. Verwendung nach Anspruch 15, wobei das hergestellte Medikament ein Impfstoff nach einem der Ansprüche 2 bis 14 ist.

17. Verfahren zur Herstellung eines Impfstoffes nach einem der Ansprüche 1 bis 14, welcher eine *Helicobacter pylori*-Infektion oder *Helicobacter pylori*assoziierte Krankheit behandelt oder verhindert, das Verfahren umfassend:
Gewinnen eines *Helicobacter pylori*-Proteins nach Anspruch 1; und
Herstellen eines Impfstoffpräparates, das das Protein oder das Derivat oder Fragment oder die Mutante davon umfasst, welche(s) geeignet ist zur Verabreichung an einen Wirt und welche(s) nach Verabreichung eine Immunantwort verursacht.

## Revendications

1. Vaccin pour le traitement ou la prophylaxie d'une infection à *Helicobacter pylori* ou d'une maladie associée à *Helicobacter pylori,* le vaccin comprenant une protéine *Helicobacter pylori* contre laquelle une réactivité immunitaire est détectée chez des individus négatifs à *Helicobacter pylori,* la protéine contenant une des protéines ou les deux protéines suivantes :
(a) une protéine *Helicobacter pylori* possédant une masse moléculaire de 18 à 19 kDa et une ou plusieurs des caractéristiques de séquence suivantes : (i) une séquence d'acides aminés N-terminale telle que répertoriée dans SEQ ID N° 2, ou une partie de celle-ci; (ii) une séquence d'acides aminés N-terminale telle que répertoriée dans SEQ ID N° 6, ou une partie de celle-ci; (iii) une séquence d'acides aminés interne telle que répertoriée dans SEQ ID N° 3, ou une partie de celle-ci; ou un dérivé, fragment ou mutant de ladite protéine *Helicobacter pylori*;
(b) une protéine *Helicobacter pylori* possédant une masse moléculaire de 24 à 25 kDa et une ou plusieurs des caractéristiques de séquence suivantes : (i) une séquence d'acides aminés N-terminale telle que répertoriée dans SEQ ID N° 1, ou une partie de celle-ci; (ii) une séquence d'acides aminés interne telle que répertoriée dans SEQ ID N° 4, ou une partie de celle-ci; ou un dérivé, fragment ou mutant de ladite protéine *Helicobacter pylori*;

2. Vaccin selon la revendication 1 dans lequel la réactivité immunologique est à base d'anticorps.

3. Vaccin selon l'une quelconque des revendications précédentes contenant un véhicule pharmaceutiquement acceptable.

4. Vaccin selon l'une quelconque des revendications précédentes en association avec un adjuvant pharmacologiquement approprié.

5. Vaccin selon la revendication 4 dans lequel l'adjuvant est l'interleukine 12.

6. Vaccin selon la revendication 4 ou 5 dans laquelle l'adjuvant est une protéine de choc thermique.

7. Vaccin selon l'une quelconque des revendications précédentes contenant au moins un autre produit pharmaceutique.

8. Vaccin selon la revendication 7 dans lequel le produit pharmaceutique est un antibiotique, de préférence l'antibiotique est choisi parmi un ou plusieurs composés parmi le métronidazole, l'amoxycilline, la tétracycline ou l'érythromycine, la clarithromycine ou le tinidazole.

9. Vaccin selon la revendication 7 ou 8 dans lequel le produit pharmaceutique contient un agent antibactérien tel que des sels de bismuth.

10. Vaccin selon l'une quelconque des revendications précédentes sous une forme appropriée pour l'administration orale.

11. Vaccin selon l'une quelconque des revendications 1 à 9 sous une forme appropriée pour l'administration intranasale.

12. Vaccin selon l'une quelconque des revendications 1 à 9 sous une forme appropriée pour l'administration intraveineuse.

13. Vaccin selon l'une quelconque des revendications 1 à 9 sous une forme appropriée pour l'administration intramusculaire.

14. Vaccin selon l'une quelconque des revendications 1 à 9 contenant un système de délivrance peptidique.

15. Utilisation de la protéine *Helicobacter pylori* telle que définie dans la revendication 1, ou d'un de ses dérivés ou fragments ou mutants, pour la préparation d'un médicament destiné au traitement ou à la prophylaxie de maladie(s) associée(s) à *Helicobacter pylori.*

16. Utilisation selon la revendication 15 dans laquelle le médicament qui est préparé est un vaccin selon l'une quelconque des revendications 2 à 14.

17. Procédé de préparation d'un vaccin selon l'une quelconque des revendications 1 à 14 qui traite ou prévient une infection à *Helicobacter pylori* ou une maladie associée à *Helicobacter pylori*, le procédé comprenant les étapes consistant à :
obtenir une protéine *Helicobacter pylori* telle que définie dans la revendication 1; et
former une préparation vaccinale composée de ladite protéine, ou d'un de ses dérivés ou fragments ou mutants, appropriée à l'administration à un hôte et qui, lorsqu'elle est administrée, déclenche une réponse immunitaire.
